# EUROPEAN PATENT APPLICATION

(11) **EP 1 870 089 A1**
(43) Date of publication of application: **26.12.2007**
(21) Application number: 05743397.1
(22) Date of filing: 15.04.2005
(51) Int. Cl.: A61K 9/02, A61K 9/16

(54) **A GYNECOLOGICAL PHARMACEUTICS-CONTAINING MICROSPHERICAL VASCULAR SUPPOSITORY OF SODIUM ALGINATE AND ITS PREPARATION METHOD**

(71) Applicant: Beijing Shengyiyao Science & Technology Development Co., Ltd, Xicheng District Beijing 100088 (CN); Fudan University Maternity Hospital, Shanghai 200011 (CN)
(72) Inventor: XU, Congjian, Shanghai 200011 (CN); LI, Xinjian, Room 211, Building B, Youyan Mansion, Beijing 100088 (CN); HONG, Hong, Room 211, Building B, Youyan Mansion, Beijing 100088 (CN); LU, Ge, Room 211, Building B, Youyan Mansion, Beijing 100088 (CN)
(74) Representative: Hart Davis, Jason
(86) International application number: PCT/CN2005/000506
(87) International publication number: WO 2006/108324

(57) **Abstract**

The present invention relates to a kind of sodium alginate microsphere vascular embolus containing gynecological drug and its preparation. The preparation of it consists of the following steps: preparing the solution of gynecological drug, sodium alginate and dilvalent metal cations such as BaCl₂, Mg Cl₂ or CaCl₂ etc. by order; mixing the gynecological drug solution and sodium alginate solution, and then mixing them with the curing solution of BaCl₂ or CaCl₂ and the like. Then the obtained is the sodium alginate microshere vascular embolus containing gynecological drug. The carrier materials of the vascular embolus according to the invention are natural extracts and have good biocompatibility and re biodegradable; the side effect of the pharmaceutic loaded by them is minimal, and it is especially suitable for myometrial gland and hysteromyoma.

## Description

### Field of the Invention

The present invention relates to a kind of sodium alginate microsphere vascular embolus containing gynecological drug and its preparation.

### Background of the Invention

Uterine fibroids and adenomyosis are the common benign disorders of female reproductive system. The high morbidity of these disorders definitely influences the quality of life of women. Medication and operation are the main therapeutic options at present. Hysterectomy is the main operation method, which is required for those with severe symptoms and elders without demand of pregnancy. Medication is suitable for the youngers with desire of pregnency, people with mild symptoms or near menopause, including Danazol, gonadotropin releasing hormone analogues (GnRHa), Mifepristone (Ru486), androgen, gestrinone, etc.

Danazol is an artificially synthesized derivative of 17-acetylene testosterone with mild androgen-like function. In vitro study has confirmed that Danazol may directly act at: (1) the hypothalamus-pituitary-ovary axis and inhibit the release of gonadotropin-releasing hormone, (2) ovary and inhibit the synthesis of ovarian hormones, (3) uterine membrane and result in atrophy of the membrane and pseudo-menopause, and (4) ectopic membrane and reduce the expression of cytochrome P450 (an aroma enzyme). However, side-effects as hepatotoxicity of Danazol limit its long-term application in clinical practice.

Gonadotropin releasing hormone analogues (GnRHa) is a kind of artificially synthesized deca-peptide compound, which can decrease the secretion of gonadotropin and ovarian hormones by means of depressing GnRHa receptor in pituitary gland, leading to atrophy of uterine membrane. Lower estrogen concentration caused by GnRHa may reduce the arterial blood supply of uterus by decreasing the vessel lumen, then reduce the size of fibroids ulteriorly. GnRHa also possesses direct action on uterine membrane. GnRHa may lead to artificial menopause so as to relieve the symptoms caused by adenomyosis, and markedly reduce the size of uterus and increase the possibility of pregnancy. GnRHa can prohibit the growth of fibroids, and pre-operative application (i.e., 3-6 months earlier) could reduce intraoperative bleeding. The main side-effect of GnRHa is menopause symptoms and osteoporosis caused by lower sex hormones, and GnRHa lacks of long-term efficacy.Higher rate of recurrence happens when GnRHa is ceased.

Androgen, Gestrinone, and Mifepristone(Ru486) act on hypothalamus-pituitary-ovary axis by means of non-rivalrous anti-estrogen, resulting in suspension of ovulation, decrease of progestogen level, decrease of arterial blood supply of uterus and atrophy of fibroids. Gestrinone is the derivative of methyltestosterone, and the mechanism is similar to that of Danazol. The side-effects as hepatotoxicity of the drugs mentioned above limit their extensive application in routine clinical practice.

With its rapid development, interventional radiology has playing an important role in modern medicine. As a result, an additional option is available for the patients with fibroids and adenomyosis besides hysterectomy and medication: interventional microinvasive therapy.

The characteristics and advantages of interventional transarterial embolotherapy for the treatment of fibroids and adenomyosis include:
1.Replacing open surgery by minimally invasive technique may relieve the patients not only physically but also psychologically;
2.Replacing whole body medication by regional embolotherapy with embolic agent/combined with infusion/biologically degradable embolic agent may:
   ①Increase drug concentration in the target region;
   ②Decrease side-effects and toxicity caused by medication;
   ③Provide double effects caused by embolization and regional drug-release;
   ④Possible pregnancy due to preservation of uterus.
3.Decrease side-effects resulted from open surgery;
4.Decrease financial burden because of shortening of hospitalization due to minimally invasive technique;
5.Increase both the social and economic benefits.

Particularly, the ages of patients suffering fibroids tend to become more and more smaller, and hysterectomy or medication is hard to satisfy the demand of preservation of uterus/ability of pregnancy.

The rapidly developed techniques of interventional therapy provide optional treatment in the field of gynecology and obstetrics. Interventional therapy is defined as superselective intraarterial infusion of embolic agent into the target region under radiological guidance such as DSA, leading to therapeutic efficacy charactered by atrophy/necrosis of the fibroids or target lesion. Among the interventional therapies for fibroids and adenomyosis, embolization of uterine artery (UAE) is the most rapidly developed one. Since 1979, UAE is widely used for the treatment of vital postpartum bleeding, ectopic pregnancy and post-operative hemorrhage, as well as malignant tumors in recent years.UAE has been proved to be safe and effective by numerous researches/publications during clinical practice of more than twenty years without ischemic necrosis of the target organ.

UAE for the treatment of fibroids and adenomyosis is used in clinical practice world-widely at present. UAE can cause regional ischemia of uterus and desquamation of uterine_membrane, which leads to the relief of the symptoms and improvement of the quality of life. 37~85% shrinkage of fibroids is also reported, which results in improvement of symptoms in 95% of the patients.

In selected patients, UAE could replace medication/hysterectomy in the patients who are aimed in relief of symptoms caused by benign tumors as fibroids.

Simple embolization with embolic agent can interrupt the blood supply of the target region (tumor), which leads to therapeutic efficacy represented by atrophy and necrosis.

Disadvantages of regional drug infusion include: uneven release of the drug and necrosis/injury of tissue caused by highly concentrated drug.

At present there is no precedent at home and abroad to take sodium alginate as drug carrier to wrap gynecological drug, and apply it in gynecological patients by vascular embolotherapy.

### Summary of the Invention

One objective of the present invention is to provide a kind of sodium alginate microsphere vascular embolus containing gynecological drug, which has good biological tolerance, less side-effects, therapeutic efficacy of embolization and medication at the same time and releases uniformly, which results in improved efficacy in treating fibroids and adenomyosis.

The said objective of the present invention is achieved by the following technique protocol.

A kind of sodium alginate microsphere vascular embolus containing gynecological drug, which is composed of sodium alginate as a drug carrier and gynecological drug which is encapsulated by said sodium alginate.

The weight ratio of said sodium alginate and said gynecological drug ranges from 1:1 to 90:1.

The said gynecological drugs include: Danazol, gonadotropin releasing hormone analogues (GnRHa), Mifepristone, Gestrinone or androgen.

The said sodium alginate microshere vascular embolus containing gynecological drug may be microgel particles or microspheres reserving in curing solution.

The said sodium alginate microshere vascular embolus containing gynecological drug may also be powdery granule.

The diameter of the said microgel particles or microspheres reserving in the curing solution ranges from 200~550µm, 400~750µm, or 600~950µm.

The diameter of the said powdery granule ranges from 100~350µm, 200~ 550µm or 400~750µm.

Another objective of the present invention is to provide preparation of sodium alginate microshere vascular embolus containing gynecological drug mentioned above.

The said objective of the present invention is achieved by the following technique protocol.

The preparation of sodium alginate microshere vascular embolus containing gynecological drug consists of the following steps:
1. Gynecological drug solution is prepared by dissolving a certain rate of gynecological drug with solvent;
2. Sodium alginate solution is prepared by dissolving a certain rate of sodium alginate;
3. Curing solution is prepared by dissolving calcium chloride, barium chloride or magnesium chloride with the concentration of 1-10%;
4. Gynecological drug solution is mixed with sodium alginate solution, then the mixture is added dropwise to curing solution under a high-voltage electrostatic droplets device to form sodium alginate microspheres or microgel particles containing gynecological drug.

The high-voltage electrostatic droplets device comprises an electrostatic device, said electrostatic device has positive and negative electrodes. The positive electrode is connected with a needle of a micro-syringe device and the negative electrode is connected with a stainless steel wires emerged in the bivalent metal cationic curing solution. The mixture solution of gynecological drug and sodium alginate in the micro-syringe device is dripped into the bivalent cationic curing solution to form microspheres.

The obtained sodium alginate microshere vascular embolus containing gynecological drug is the microsphere reserving in the curing solution, which is called as wet-microsphere, the diameter can range from 200~550µm, 400~ 750µm or 600~950µm. Wet-microsphere of 300~550µm is required for the treatment of adenomyosis, 500~750µm for uterine fibroids, and 700~950µm for the patients with demand of pregnancy.

The obtained sodium alginate microshere vascular embolus containing gynecological drug is decanted, then the lower microgel particles are put into the oven and kept in a sealed condition. The obtained powdery granules are called dry microspheres. The diameters of which may range from 100~350µm, 200~550µm or 400~750µm. Dry microsphere of 100~350µm is required for the treatment of adenomyosis, 200~550µm for uterine fibroids, and 400~ 750µm for the patients with demand of pregnancy.

The third objective of the present invention is to provide application of the sodium alginate microshere vascular embolus containing gynecological drug mentioned above.

The said objective of the present invention is achieved by the following technique protocol.

By adopting intervention radiotherapy or intervention ultrasound, the duct is inserted into feeding artery of target organ to conduct arteriography. The selected diameter of embolus microsphere is determined according to the impression of arteriography. Aseptically open the bottle and leave it until the microspheres are settled at the bottom of the bottle. Remove the curing solution by a syringe then add an equal amount of normal saline to wash the microspheres three times, or remove the curing solution by a syringe then add an equal amount of normal saline, pour normal saline and microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.

If the sodium alginate microshere vascular embolus containing gynecological drug is powdery granule, then the dry microsphere reserving in sealed container are dissolved in normal saline to swell (wet microsphere), then appropriate amount or diluted contrast medium is added to mix evenly (leaving microsphere thoroughly floating in the contrast medium), and then the mixture is infused slowly or slowly in multiple times in the vessel of the affected portion to embolism ultraselectively by duct under the monitor of image documentation equipment (Over-embolization is absolutely prohibited.) till the flow rate of contrast medium is obviously reduced, i.e. embolism is finished. Arteriography is conducted again to judge the effect of embolism.

The following is the further illustration of the present invention in embodiments, but it does not mean any limitation for the protection range of it.

### Detailed Description of the Invention

### Example 1

### Preparation of sodium alginate microsphere vascular embolus containing Danazol

### 1. Preparation before wrapping

① Disposal of glass utensil:
   The clean glass utensil is dried then kept in oven at 300 °C for 3 hours (the heat source is removed till the bacteria are killed);
② Preparation of Danazol solution:
   1.5 kg Danazol available in market is weighed and placed in the glass utensil mentioned above, propylene glycol is added dropwise till Danazol is completely dissolved to make a Danazol solution;
③ Preparation of sodium alginate solution:
   2.0 kg sodium alginate available in market is weighed and placed in the glass utensil, normal saline is added while stirring till sodium alginate is completely dissolved;
④ Preparation of 1% CaCl₂ solution;
⑤ The Danazol solution is mixed with sodium alginate solution;
⑥ The mixed solution is extracted by aseptic syringe and added into the calcium chloride solution under the high-voltage electrostatic droplet device. The sodium alginate microsphere containing Danazol settles at the bottom of the glassware with diameter of 200~550µm.

The upper solution is decanted and the lower microgel particles are put into the oven and keeped in a sealed condition. The diameters of dry microspheres are 100~350µm. Prior to use, the dry microspheres should be rehydrated with normal saline for a couple of minutes.

Alternatively, the wet-microspheres can be used directly after the decanter of the upper solution and washed twice.

As for patients with myometrial gland, by adopting intervention radiotherapy or intervention ultrasound, insert the duct into feeding artery of target organ to conduct arteriography. Paclitaxel-containing sodium alginate microsphere with diameter ranging from 200-550µm is selected according to the impression of arteriography. Try to use micro-duct to carry out ultraselective embolus with aseptic operation. Extract CaCl₂ solution from the bottle using injector and add in equal volume normal saline to rinse Danazol-containing sodium alginate microsphere (wet ball) for 3 times, or extract the described CaCl₂ solution from the bottle and add in equal volume normal saline, pour normal saline and

microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it into the foci slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.

### Example 2

### Preparation of sodium alginate microsphere vascular embolus containing Gonadotropin releasing hormone analogues (GnRHa)

### 1. Preparation before wrapping

① Disposal of glass utensil:
   The clean glass utensil is dried then kept in oven at 300°C for 3 hours (the heat source is removed till the bacteria are killed);
② Preparation of Gonadotropin releasing hormone analogues (GnRHa) solution:
   2 kg Gonadotropin releasing hormone analogues (GnRHa) available in market is weighed and placed in the glass utensil mentioned above, normal saline is added dropwise till Gonadotropin releasing hormone analogues (GnRHa) is completely dissolved to make a Gonadotropin releasing hormone analogues (GnRHa) solution;
③ Preparation of sodium alginate solution:
   20 kg sodium alginate available in market is weighed and placed in the glass utensil, normal saline is added while stirring till sodium alginate is completely dissolved;
④ Preparation of 1% MgCl₂ solution;
⑤ The Gonadotropin releasing hormone analogues (GnRHa) solution is mixed with sodium alginate solution;
⑥ The mixed solution is extracted by aseptic syringe and added into the MgCl₂ solution under the high-voltage electrostatic droplet device. The sodium alginate microsphere containing Gonadotropin releasing hormone analogues (GnRHa) settles at the bottom of the glassware with diameter of 200~550µm.

The upper solution is decanted and the lower microgel particles are put into the oven and keeped in a sealed condition. The diameters of dry microspheres are 100~350µm. Prior to use, the dry microspheres should be rehydrated with normal saline for a couple of minutes.

Alternatively, the wet-microspheres can be used directly after the decanter of the upper solution and washed twice.

As for patients with hysteromyoma, by adopting intervention radiotherapy or intervention ultrasound, insert the duct into feeding artery of target organ to conduct arteriography. Gonadotropin releasing hormone analogues (GnRHa)-containing sodium alginate microsphere with diameter ranging from 200-550µm is selected according to the impression of arteriography. Try to use micro-duct to carry out ultraselective embolus with aseptic operation. Extract CaCl₂ solution from the bottle using injector and add in equal volume normal saline to rinse paclitaxel-containing sodium alginate microsphere (wet ball) for 3 times, or extract the described MgCl₂ solution from the bottle and add in equal volume normal saline, pour normal saline and microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it into the foci slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.

### Example 3

### Preparation of sodium alginate microsphere vascular embolus containing androgen

### 1. Preparation before wrapping

① Disposal of glass utensil:
   The clean glass utensil is dried then kept in oven at 300°C for 3 hours (the heat source is removed till the bacteria are killed);
② Preparation of androgen solution:
   2 kg androgen available in market is weighed and placed in the glass utensil mentioned above, acetone is added dropwise till androgen is completely dissolved;
③ Preparation of sodium alginate solution:
   50kg sodium alginate available in market is weighed and placed in the glass utensil, normal saline is added while stirring till sodium alginate is completely dissolved;
④ Preparation of 6% BaCl₂ solution;
⑤ The androgen solution is mixed with sodium alginate solution;
⑥ The mixed solution is extracted by aseptic syringe and added into the BaCl₂ solution under the high-voltage electrostatic droplet device. The sodium alginate microspheres containing androgen settles at the bottom of the glassware with diameters of 600~950µm.

The upper solution is decanted and the lower microgel particles are put into the oven and kept in a sealed condition. The diameters of dry microspheres are 400~750µm. Prior to use, the dry microspheres should be rehydrated with normal saline for a couple of minutes.

Alternatively, the wet microspheres can be used directly after the decanter of the upper solution and washed twice.

As for patients with hysteromyoma, by adopting intervention radiotherapy or intervention ultrasound, insert the duct into feeding artery of target organ to conduct arteriography. androgen -containing sodium alginate microsphere with diameter ranging from 600-750µm is selected according to the impression of arteriography. Try to use ultraselective micro-duct to carry out embolus with aseptic operation while using. Extract CaCl₂ solution from the bottle using injector and add in equal volume normal saline to rinse androgen -containing sodium alginate microsphere (wet ball) for 3 times, or extract the described BaCl₂ solution from the bottle and add in equal volume normal saline, pour normal saline and microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it into the foci slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.

### Example 4

### Preparation of sodium alginate microsphere vascular embolus containing gestrinone

### 1. Preparation before wrapping

① Disposal of glass utensil:
   The clean glass utensil is dried then kept in oven at 300°C for 3 hours (the heat source is removed till the bacteria are killed);
② Preparation of gestrinone solution:
   2 kg androgen available in market is weighed and placed in the glass utensil mentioned above, propylene glycol is added dropwise till gestrinone is completely dissolved;
③ Preparation of sodium alginate solution:
   150kg sodium alginate available in market is weighed and placed in the glass utensil, normal saline is added while stirring till sodium alginate is completely dissolved;
④ Preparation of 6% CaCl₂ solution;
⑤ The gestrinone solution is mixed with sodium alginate solution;
⑥ The mixed solution is extracted by aseptic syringe and added into the calcium chloride solution under the high-voltage electrostatic droplet device. The sodium alginate microspheres containing androgen settles at the bottom of the glassware with diameters of 500~750µm.

The upper solution is decanted and the lower microgel particles are put into the oven and kept in a sealed condition. The diameters of dry microspheres are 300~500µm. Prior to use, the dry microspheres should be rehydrated with normal saline for a couple of minutes.

Alternatively, the wet microspheres can be used directly after the decanter of the upper solution and washed twice.

As for patients with hysteromyoma, by adopting intervention radiotherapy or intervention ultrasound, insert the duct into feeding artery of target organ to conduct arteriography. gestrinone-containing sodium alginate microsphere with diameter ranging from 600-750µm is selected according to the impression of arteriography. Try to use ultraselective micro-duct to carry out embolus with aseptic operation while using. Extract CaCl₂ solution from the bottle using injector and add in equal volume normal saline to rinse androgen -containing sodium alginate microsphere (wet ball) for 3 times, or extract the described BaCl₂ solution from the bottle and add in equal volume normal saline, pour normal saline and microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it into the foci slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.

### Example 5

### Preparation of sodium alginate microsphere vascular embolus containing mifepristone

### 1. Preparation before wrapping

① Disposal of glass utensil:
   The clean glass utensil is dried then kept in oven at 300°C for 3 hours (the heat source is removed till the bacteria are killed);
② Preparation of mifepristone solution:
   2 kg androgen available in market is weighed and placed in the glass utensil mentioned above, propylene glycol is added dropwise till gestrinone is completely dissolved;
③ Preparation of sodium alginate solution:
   10kg sodium alginate available in market is weighed and placed in the glass utensil, normal saline is added while stirring till sodium alginate is completely dissolved;
④ Preparation of 6% MgCl₂ solution;
⑤ The gestrinone solution is mixed with sodium alginate solution;
⑥ The mixed solution is extracted by aseptic syringe and added into the MgCl₂ solution under the high-voltage electrostatic droplet device. The sodium alginate microspheres containing androgen settles at the bottom of the glassware with diameters of 550~700µm.

The upper solution is decanted and the lower microgel particles are put into the oven and kept in a sealed condition. The diameters of dry microspheres are 250~550µm. Prior to use, the dry microspheres should be rehydrated with normal saline for a couple of minutes.

Alternatively, the wet microspheres can be used directly after the decanter of the upper solution and washed twice.

As for patients with hysteromyoma, by adopting intervention radiotherapy or intervention ultrasound, insert the duct into feeding artery of target organ to conduct arteriography. mifepristone-containing sodium alginate microsphere with diameter ranging from 600-750µm is selected according to the impression of arteriography. Try to use ultraselective micro-duct to carry out embolus with aseptic operation while using. Extract CaCl₂ solution from the bottle using injector and add in equal volume normal saline to rinse androgen -containing sodium alginate microsphere (wet ball) for 3 times, or extract the described BaCl₂ solution from the bottle and add in equal volume normal saline, pour normal saline and microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it into the foci slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.

### Industrial Application

Sodium alginate, used as a drug carrier in this invention is natural extracts, which is a kind of polysaccharide sodium salt composed byβ-D-mannitol and α-L-gulose extracted from natural brown algae. As a kind of linear macromolecule, its molecular weight is 50,000-100,000 Dalton. It has high hydrophilicity and can be easily dissolved in water forming viscous colloid. Cross-link and solidification among macromolecular links may occur under the action of calcium ion. It can be processed into solid spherical or spherical like microsphere in different sizes according to clinical necessity. This kind of microsphere has good biocompatibility. In vivo the living organism, calcium ion may be gradually educed, and microsphere is nontoxicly degraded within 3-6 months due to molecular chain scission. No debris is produced during degradation, and it may also result in the eternal vascular embolism in target organs (When embolus remains in the vessel as long as 2 months, the intravascular thrombus may form and achieve the objective of eternal embolism.), thus to achieve the goal of treatment. In practical operation, using this kind of "biological multifunctional microsphere" embolism materials, by physical clogging tumor or arteriole vessel around the treated portion, may cause vascular closure, block blood supply and nutrients of the tissue in that portion, thus to induce atrophy and necrosis due to 1 ischemia and hypoxia. Meanwhile, it may provide advantageous condition for operation by reducing blood supply of target organs. Therefore, it shows dual therapeutic effect of embolism and drugs by taking this microsphere as drug carriers in treatment of gynecological diseases to slowly release to the tissue of local foci in a timing, positioning, and orientation way, thus to improve therapeutic efficacy greatly, especially for myometrial gland and hysteromyoma.

The sodium alginate microsphere vascular embolus containing gynecological drug for uterine artery embolization (UAE) are indicated for treating fibroids and adenomyosis by regional release of drug and interruption of blood supply. The presented invention is charactered by the combination of gynecological drugs having direct therapeutic efficacy, such as Danazol, GnRHa, Mifepristone, Gestrinone, androgen, and sodium alginate(KMG), a biologically degradable drug-containing microsphere as an embolic agent for the treatment of fibroids and adenomyosis by means of intraarterial superselective embolization of uterine artery under radiological guidance is obtained. Release of the drugs lasts for about 2 months, leading to double therapeutic efficacy by regional medication and ischemia. Gradual release of the drugs could not only increase the regional drug concentration by first effect of drug but also reduce side-effect. Meanwhile, some of the drugs released into the circulation system could provide long-term effect by accommodate the gonad axis.

## Claims

1. A kind of sodium alginate microsphere vascular embolus containing gynecological drug, which is comprised of sodium alginate as a drug carrier and gynecological drug, said gynecological drug is enwrapped by said sodium alginate.

2. A kind of sodium alginate microsphere vascular embolus containing gynecological drug as in claim 1, wherein, the weight ratio of said sodium alginate and said gynecological drug ranges from 1:1 to 90:1.

3. A kind of sodium alginate microsphere vascular embolus containing gynecological drug as in claim 1, wherein, said gynecological drug include Danazol, GnRHa, Mifepristone, Gestrinone, or androgen.

4. A kind of sodium alginate microsphere vascular embolus containing gynecological drug as in claim 1, wherein, said sodium alginate microshere vascular embolus containing gynecological drug may be microgel particles or microspheres reserving in curing solution.

5. A kind of sodium alginate microsphere vascular embolus containing gynecological drug as in claim 1, wherein, said sodium alginate microshere vascular embolus containing gynecological drug may also be powdery granule.

6. A kind of sodium alginate microsphere vascular embolus containing gynecological drug as in claim 4, wherein, the diameter of the said microgel particles or microspheres reserving in the curing solution ranges from 300-550µm or 500-750µm or 700-950µm.

7. A kind of sodium alginate microsphere vascular embolus containing gynecological drug as in claim 5, wherein, the diameter of the said powdery granule ranges from 100-350µm or 200-550µm or 400-750µm.

8. The preparation of sodium alginate microsphere vascular embolus containing gynecological drug consists of the following steps:
(1) gynecological drug solution is prepared by dissolving a certain rate of gynecological drug with solvent;
(2) Sodium alginate solution is prepared by dissolving a certain rate of sodium alginate;
(3) Curing solution is prepared by dissolving calcium chloride, barium chloride or magnesium chloride with the concentration of 1-10%;
(4) gynecological drug solution is mixed with sodium alginate solution, then the mixture is added dropwise to said curing solution under a high-voltage electrostatic droplets device to form sodium alginate microspheres or microgel particles containing gynecological drug.

9. The preparation of A kind of sodium alginate microsphere vascular embolus containing gynecological drug as in claim 8, wherein, The high-voltage electrostatic droplets device comprises an electrostatic device, said electrostatic device has positive and negative electrodes, the positive electrode is connected with a needle of a micro-syringe device and the negative electrode is connected with a stainless steel wires emerged in the bivalent metal cationic curing solution, the mixture solution of gynecological drug and sodium alginate in the micro-syringe device is dripped into the bivalent cationic curing solution to form microspheres.

10. The preparation of A kind of sodium alginate microsphere vascular embolus containing gynecological drug as in claim 8 or 9, wherein, said sodium alginate microsphere is dried to obtain powdery granule.

11. The application of said A kind of sodium alginate microsphere vascular embolus containing gynecological drug as in any one of claims 1 to 7, wherein the sodium alginate microsphere vascular embolus containing gynecological drug is superselectively embolized in the vein of the disease part using micro-catheter under angiography device.
